# EUROPEAN PATENT APPLICATION

(11) **EP 4 265 184 A1**
(43) Date of publication of application: **25.10.2023**
(21) Application number: 22169611.5
(22) Date of filing: 24.04.2022
(51) Int. Cl.: A61B 5/1495, A61B 5/145, A61B 5/1455, A61B 5/00

(54) **TEMPERATURE CALIBRATION**

(71) Applicant: Indigo Diabetes N.V., 9052 Gent (BE)
(72) Inventor: The designation of the inventor has not yet been filed
(74) Representative: Winger

(57) **Abstract**

A sensor system for sensing at least one compound. The sensor system comprises a sensing device comprising a sensor for capturing spectroscopic information regarding at least one compound and a temperature sensor for capturing temperature information regarding the analyte. The sensor system is being adapted for deriving temperature information from the spectroscopic information and for controlling and/or adapting the sensor system based on a comparison of the temperature information derived from the spectroscopic information and the temperature information captured from the temperature sensor.

## Description

### Technical field of the invention

The present invention relates to the field of sensor devices, More particularly, the present invention relates to a sensor device, such as for example an implantable sensor device for measuring one or more endogenous and/or exogeneous compounds, such as for example metabolites like e.g. glucose, in the body of a living creature.

### Background of the invention

Sensor devices are ever more used as an aid in diagnosing or monitoring of illnesses or medical conditions and lifestyle health monitoring/applications such as for example monitoring of sports, diets, endurance, etc.. In particular wearable and implantable sensor devices may enable continuous monitoring and high convenience to the end user. Such sensor devices, especially when implanted subcutaneously, require an accurate calibration and control in order to ensure accurate sensing, e.g. sensing of endogenous and/or exogenous compounds such as for example metabolites.

Accurate calibration of implantable sensor devices often requires complex procedures whereby additional measurements are required using further systems and or equipment, such as for example requiring capturing and analysis of a fluid sample from the patient who intends to use the implantable sensor using a further external system and calibrating the implantable sensor device based on the information obtained with the further external system. Aside from being a hassle, the process also can lead to erroneous calibration, e.g. because errors are made with respect to timing, because the hands were dirty during the calibration process, etc..

Sensor devices in general and implantable specifically may be subject to drift and deterioration. Since these sensor devices are implanted, it is advantageous to avoid changes resulting in sensor devices that lose accuracy and to lengthen the lifetime of these sensor devices for as long as possible, thus avoiding the need for replacement.

There is still a need for optimising calibration, accuracy and/or lifetime of sensing systems using an at least partially implantable sensing device.

### Summary of the invention

It is an object of the present invention to provide good sensing systems. It is an advantage of embodiments of the present invention that devices are obtained to have a good accuracy. The latter may especially be advantageous for implantable or partially implantable devices, although embodiments are not limited thereto.

It is an advantage of embodiments of the present invention that sensing systems are provided that can be calibrated for initial use without the need of other calibration measurements to be performed using further systems or equipment. It is for example an advantage that calibration of sensing systems can be performed without the need for measurements on a separately collected sample of the individual in who the implantable device is at least partially implanted, such as for example on a blood sample.

It is an advantage of embodiments of the present invention that devices, e.g. implantable or partially implantable devices, are obtained having a prolonged lifetime.

It is an advantage of embodiments of the present invention that devices, e.g. implantable or partially implantable devices, are obtained that can be individually tailored to the conditions of the user.

The above objective is accomplished by a method and apparatus according to the present invention.

The present invention relates to a sensor system for sensing at least one compound - e.g. in analyte of a living creature, but not limited thereto -, the sensor system comprising a sensing device. The analyte may be tissue, ISF, blood, urine, saliva, tears or another bodily fluids or substances. The sensing device comprises
a sensor for capturing spectroscopic information regarding at least one compound in an analyte, and
a temperature sensor for capturing temperature information regarding the analyte.

In some embodiments the temperature is measured with a sensor on or close to a substrate which is in contact with the analyte, the sensed temperature thus being representative of a temperature of the analyte. Due to integration and/or placement of a temperature sensor close to or in a substrate in contact with the analyte, the temperature may be representative of the ambient analyte. Except for local temperature fluctuations during measurement, when not taking spectral data or communicating or charging, the temperature sensor thus may measure the ambient temperature, which is the analyte temperature.

The sensor system furthermore is being adapted for deriving temperature information from the spectroscopic information and for controlling and/or adapting the sensor system based on a comparison of the temperature information derived from the spectroscopic information and the temperature information captured from the temperature sensor.

It is an advantage of embodiments of the present invention that based on temperature information, the accuracy and/or proper operation of the sensing system can be controlled.

The sensor system may be configured for performing, based on said comparison, an initial calibration of the sensor system after implantation or for performing a recalibration of the sensor system when in use. It is an advantage of embodiments of the present invention that based on the temperature information calibration of the sensor system can be performed. It is an advantage that in this way, calibration of the sensor system can be performed, without the need for another calibration measurement, such as for example measurement of a separately collected blood sample or alike. It is an advantage of at least some embodiments of the present invention that the calibration of the sensor system can be updated. The comparison of the temperature information thus may allow for guaranteeing or restoring a minimal accuracy of the sensor system. It is an advantage of embodiments of the present invention that sensor systems that have run out of their working range and therefor would become not operational anymore can be recalibrated to be in an operational range again, and thus can further be used.

The sensor system may be adapted for performing an initial calibration based on said comparison and taking into account a modelling of the sensor system behaviour after implantation. It is an advantage of embodiments of the present invention that the initial calibration takes into account modelling of the temperature behaviour of the sensor system after implantation of the sensor system.

The spectroscopic information from which the temperature information is derived may be information obtained using chemometrics. The temperature information may be based on multivariate techniques, such as for example multivariate calibration techniques and/or multivariate analysis. The temperature information may for example be based on partial least square techniques and/or on principal component analysis (PCA) techniques on UV/VIS NIR spectroscopy.

The calibration may be a personalised calibration of the sensor system fit to the individual user, based on said comparison. It is an advantage of embodiments of the present invention that calibration of the sensing system can be adjusted to the user, i.e. can be adjusted to take into account influences caused by the individual living creature for whom it is used. This allows for a personalised tailoring of the sensing system to the user, thus resulting in more accurate use of the sensing system.

The sensor system may be adapted for, based on said comparison, identifying a drift e.g. caused by drift of one or more components of the sensor system or drift of the sensor system caused by external causes such as encapsulation of the at least partially implantable sensing device.

The sensing device may be an at least partially implantable sensing device or may be fully implantable, such as for example fully implantable subcutaneous. Alternatively, the at least partially implantable sensing device may be for example a needle based sensor whereby a needle is inserted in the patient but whereby another part of the at least partially implantable sensing device is maintained close to but outside the body of the living creature.

It is an advantage of embodiments of the present invention that information, such as for example sensing results, can be blocked from being displayed, in case the system seems defect or seems not operating in a correct manner.

In some embodiments, the system may be configured for performing one or more steps of the following algorithm :
- Measuring the temperature using one or multiple temperature sensor(s)
- Deriving temperature from the spectroscopic information measured with the spectrometer, e.g. based on a spectral chemometrics model
- Comparing the temperature measured with the temperature sensor and the temperature derived from the spectroscopic information to see whether both temperatures are equal or within a given or predetermined specification.
- If the comparison indicates that both temperatures are equal or within a given or predetermined specification, measurement information may be provided to the user.
- If the comparison indicates that the temperatures differ more than according to a given or predetermined specification, the chemometrics model may be adapted to bring the model in calibration again and the sensing measurement may be repeated so accurate information can be provided to the user.
- If recalibration is not possible, the system may show an error message indicating the system cannot be used.

The sensing device may comprise a processor for performing said comparison. It is an advantage of embodiments of the present invention that processing of the information can be performed in the sensing device, allowing for fast and immediate correction where required.

The sensor system may comprise an external control device for controlling the at least partially implantable sensing device, the external control device comprising a processor for performing said comparison. It is an advantage of embodiments of the present invention that processing can be performed on an external control device, resulting in a reduced battery consumption of the sensing device, e.g. the at least partially implantable sensing device.

The external control device may be any of a dedicated external control device or a mobile apparatus, such as for example a mobile phone, comprising software for controlling the sensing device and for performing said comparison.

The sensor system may be configured to communicate with a cloud-based processor for performing said comparison or the sensor system may comprise a cloud-based processor. It is an advantage of at least some embodiments of the present invention that the processing may be performed in a cloud-based processor. It is an advantage of at least some embodiments of the present invention that the cloud-based processor may take into account information of both the sensor system concerned and other information, such as for example statistical information obtained from other sensor systems.

The temperature sensor may be a bandgap temperature sensor, e.g. a silicon bandgap temperature sensor. It is an advantage of embodiments of the present invention that use can be made of a commonly known temperature sensor, such as the silicon bandgap temperature sensor. It is an advantage of embodiments of the present invention that the temperature sensor can be easily integrated in an integrated circuit, as often is present in an at least partially implantable sensing device. One particular example of a temperature sensor that could be used, embodiments not being limited thereto, is the Sensirion AG's humidity/temperature sensing platform IC SHT35. In alternative embodiments, also other types of temperature sensors may be used such as for example a thermocouple or any other temperature sensor providing temperature information in the relevant temperature range. In embodiments according to the present invention, temperature may for example be measured in the range 20°C to 60°C, for example in the range 30°C to 50°C, for example in the range 33°C to 45°C. In at least some embodiments, the sensing device may be a photonic based sensing device.

The sensor for capturing spectroscopic information may be integrated in a silicon photonics integrated circuit and configured for performing spectral absorption, reflection or transmission measurements for detecting spectral information, e.g. absorption bands, of the at least one compound. The at least one compound may be one or more of acetate, amino acids, ammonia, creatinine, ethanol, glucose, homocysteine, ketones, lactate, lipids, phenyl sulfate, proponic acid, urea, uric acid, pyruvate or vitamin B11 and/or B12.

In one aspect, the present invention also relates to a method for using a sensor system for sensing at least one compound in a living creature's analyte, the method comprising capturing spectroscopic information using a sensor of a sensing device and deriving temperature information from said spectroscopic information and capturing temperature information regarding the analyte using a temperature sensor positioned on or near the analyte. The method further comprises controlling and/or adapting the sensor system based on a comparison of the temperature information derived from the spectroscopic information and the temperature information captured from the temperature sensor.

The method may further comprise comparing the temperature information derived from the spectroscopic information and the temperature information from the temperature sensor and deriving based thereon calibration or recalibration information from the sensor system.

Said comparing and/or said deriving may be performed in an implantable part of the sensor system, on a processor in an external device such as for example a dedicated external device or a mobile apparatus, e.g. mobile phone, configured for controlling an at least partially implantable device, or in a cloud-based processor.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantial new and novel improvements, including departures from prior practices, resulting in the provision of more efficient, stable and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

FIG. 1 is a schematic representation of a sensor system according to an embodiment of the present invention.
FIG. 2 is an illustration of how spectroscopic information from a sensor system according to embodiments of the present invention can be used for deriving temperature information, according to an embodiment of the present invention.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. The term "comprising" therefore covers the situation where only the stated features are present and the situation where these features and one or more other features are present. The word "comprising" according to the invention therefore also includes as one embodiment that no further components are present. Thus, the scope of the expression "a device comprising means A and B" should not be interpreted as being limited to devices consisting only of components A and B. It means that with respect to the present invention, the only relevant components of the device are A and B.

Similarly, it is to be noticed that the term "coupled", also used in the claims, should not be interpreted as being restricted to direct connections only. The terms "coupled" and "connected", along with their derivatives, may be used. It should be understood that these terms are not intended as synonyms for each other. Thus, the scope of the expression "a device A coupled to a device B" should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

The invention will now be described by a detailed description of several embodiments of the invention. It is clear that other embodiments of the invention can be configured according to the knowledge of persons skilled in the art without departing from the technical teaching of the invention, the invention being limited only by the terms of the appended claims.

In a first aspect, the present invention relates to a sensor system for sensing at least one compound in a living creature's analyte. The sensor system may for example be used to monitor the concentration of compound(s) in an analyte of a living creature, e.g., of the concentration of glucose present in tissue, interstitual fluid, urine, saliva or blood of the user. It is to be noted that the compound may be one or more of acetate, amino acids, ammonia, creatinine, ethanol, glucose, homocysteine, ketones, lactate, lipids, phenyl sulfate, proponic acid, urea, uric acid, pyruvate, vitamin B11 and/or B12, and water, the present invention not being limited thereto. The sensor system comprises a sensing device which may be not implanted, which may be at least partially implantable in the body of the living creature or which may be fully implantable in the body of the living creature, such as for example be fully subcutaneous implanted. The sensor may in one example also be a needle based device, whereby the needle is inserted in the body of the living creature and comprises the sensor based portion, but a remaining portion is maintained outside the body of the living creature.

The sensing device according to embodiments of the present invention comprises a sensor for capturing spectroscopic information regarding at least one compound and a temperature sensor for capturing temperature information regarding the analyte.

The sensor system furthermore is adapted for deriving temperature information from the spectroscopic information and for controlling and/or adapting the sensor system based on a comparison of the temperature information derived from the spectroscopic information and the temperature information captured from the temperature sensor.

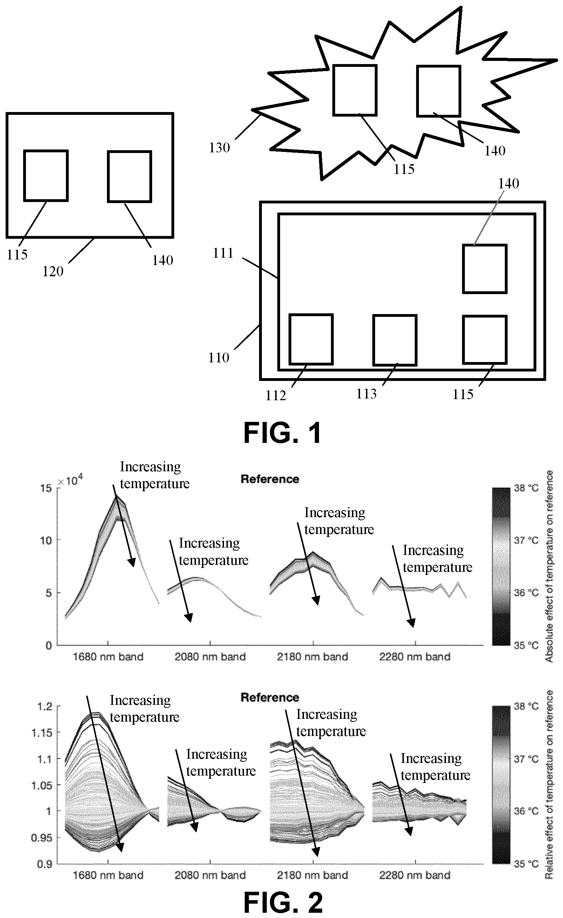

Standard and optional features of embodiments of the present invention will further be discussed with reference to an exemplary embodiment, a schematic representation thereof being shown in FIG. 1.

FIG. 1 illustrates a schematic representation showing a sensor system 100 with a sensing device 110 that is at least partially implantable in the body of the living creature. For reasons of clarity, some elements are not shown. The at least partially implantable sensing device 110 according to embodiments of the present invention comprises a substrate 111 with a sensor 112 for capturing spectroscopic information regarding at least one compound. Alternatively, the sensor 112 could be not integrated on a substrate. In embodiments wherein the sensor 112 is integrated, the substrate 111 may be or may be part of a semiconductor platform, e.g., an integrated circuit and/or a photonic integrated circuit, which may be made, for example, of silicon, silicon-oxide, silicon-carbide, or silicon-nitride or other typical materials of the semiconductor industry. The sensor 112 in the present example comprises a light emitter for emitting light and a light receiver adapted for receiving the light from the light emitter, typically after transmission through or reflection by a material to be analysed. The sensor 112 typically provides spectroscopic information. The sensor 112 thus typically is an optical, e.g., spectroscopic, sensor element. According to embodiments of the present invention, the sensing device 110 furthermore comprises a temperature sensor 113 positioned on or near the substrate 111 for capturing temperature information regarding the substrate. One example of such a temperature sensor is a bandgap temperature sensor, e.g. a silicon bandgap temperature sensor. Such sensors are commonly known temperature sensors, such as the silicon bandgap temperature sensor, which often are integrated in substrates such as integrated circuits or photonics circuits. Nevertheless, it is to be noted that the temperature sensor may also be a different type of temperature sensor, such as for example a thermocouple. More generally any other temperature sensor providing temperature information in the relevant temperature range may be used. In embodiments according to the present invention, temperature may for example be measured in the range 20°C to 60°C, for example in the range 30°C to 50°C, for example in the range 33°C to 45°C. One particular example of a temperature sensor that could be used, embodiments not being limited thereto, is the Sensirion AG's humidity/temperature sensing platform IC SHT35 or similar.

According to embodiments of the present invention, the sensor system 100 furthermore is adapted for deriving temperature information from the spectroscopic information. The sensor system 100 may therefore comprise a processor 115 on the at least partially implantable sensing device 110, on an external control device 120 and/or in the cloud 130. Such a processor 115 may be a conventional hardware processor, a dedicated processor or may be based on a general processor. It also may be implemented as a software processor.

As indicated above, an external control device 120 may be present for controlling the sensing device and or for reading out information from the sensing device. Data communication may be arranged between the sensing device and the external control device.

By way of illustration, embodiments of the present invention not being limited thereto, an example of how temperature information can be derived from the spectroscopic information is shown in FIG. 2. FIG. 2 illustrates the effect (upper graph) and the normalised effect (lower graph) of temperature on different wavelength ranges, e.g. a wavelength band around 1680nm, around 2080nm, around 2180nm and around 2280nm. One or more of these or other regions may be used for deriving a temperature from the measured spectroscopic information, which then can be compared with temperature data obtained with the temperature sensor.

The spectroscopic information from which the temperature information is derived may be information obtained using chemometrics. Chemometrics is a statistical method, applicable to e.g. NIR spectroscopy, UV spectroscopy, VIS spectroscopy or a combination thereof, to extract relevant information regarding components in the spectra. Such components may include chemical components, such as for example glucose, but also temperature. In embodiments of the present invention, the techniques thus at least can be used for deriving a temperature from the spectrum. Examples of how chemometric methods are used can for example be found in Biancolillo et al., Frontiers in Chemistry, 6, p. 576, 2018 or in Deming et al., Chemometrics: a textbook, Elsevier, 1988.

The temperature information may be based on multivariate techniques, such as for example multivariate calibration techniques and/or multivariate analysis. The temperature information may for example be based on partial least square techniques and/or on principal component analysis (PCA) techniques on UV/VIS NIR spectroscopy.

Partial least squares (PLS) analysis may be used as a quantitative chemometric technique. Examples thereof are described in Geladi et al., Analytica chimica acta, 185, pp 1 - 17, 1986 or in Bro et al. Journal of Chemometrics, 23, p 69, 2009. To develop a robust chemometric model, first a calibration ("training") is done. A robust calibration dataset is obtained through a careful design of experiments (DoE) for measuring different relevant levels of temperature in combination with different relevant levels of possible interferents. By establishing a linear relationship between the measured data from the sensor and the known temperature reference data, the PLS technique finds an optimal model to calculate the reference value, in this case, temperature. In addition thereto, the technique can further be used to identify chemical components such as for example but not limited to glucose.

Afterwards an independent validation with this model is done to define ("predict") the temperature of samples with known or unknown (but not included in the training dataset) temperature defined using another DoE.

As described above the development of a chemometric model consist out of 2 parts: training (calibration) and validation. In some embodiments of the present invention, the calibration and validation are done in vitro in the first instance and later transferred to an in vivo model using a chemometric model transfer. An example of how such a transfer can be applied is described in Brouckaert et al. in Talenta, 179, p 386, 2018.

Exemplary parts of spectroscopic information based on which chemometrics can for example be applied is shown in FIG. 2.

The sensor system 100 furthermore may be adapted for controlling and/or adapting the sensor system based on a comparison of the temperature information derived from the spectroscopic information and the temperature information captured from the temperature sensor. The sensor system 100 may therefore comprise a controller 140 which may be implemented in the sensing device 110, the external control device 120 and/or in the cloud 130. Controlling the sensor system and or adapting the sensor system may for example comprises one or more of the following actions.
- performing, based on said comparison, an initial calibration of the sensor system after implantation. The latter may for example be performed taking into account modelling of the behaviour, e.g. temperature behaviour, of the at least partially implantable device
- performing a recalibration the sensor system, e.g. based on identification of drift of one of the components in the sensing device or drift caused externally to the sensing device.

In some embodiments, the system may be configured for performing one or more steps of the following algorithm:
- Measuring the temperature using the temperature sensor
- Deriving temperature from the spectroscopic information measured with the spectrometer, e.g. based on a spectral chemometrics model
- Comparing the temperature measured with the temperature sensor and the temperature derived from the spectroscopic information to see whether both temperatures are equal or within a given or predetermined specification.
- If the comparison indicates that the temperatures are equal or within a given or predetermined specification, measurement information may be provided to the user.
- If the comparison indicates that the temperatures differ more than according to a given or predetermined specification, the chemometrics model may be adapted to bring the model in calibration again and the sensing measurement may be repeated so accurate information can be provided to the user.
- If recalibration is not possible, the system may show an error message indicating the system cannot be used.

The above steps or part thereof may be implemented as algorithm.

In a second aspect, the present invention relates to a method for using a sensor system for sensing at least one compound in a living creature's analyte, the method comprising capturing spectroscopic information using a sensor of a sensing device and deriving temperature information from said spectroscopic information and capturing temperature information regarding the analyte using a temperature sensor. The method further comprises controlling and/or adapting the sensor system based on a comparison of the temperature information derived from the spectroscopic information and the temperature information captured from the temperature sensor. Further method steps may correspond with the functionality provided by standard and optional features of systems as described in the first aspect.

It is to be understood that although preferred embodiments, specific constructions and configurations, as well as materials, have been discussed herein for devices according to the present invention, various changes or modifications in form and detail may be made without departing from the scope of this invention. Steps may be added or deleted to methods described within the scope of the present invention.

## Claims

1. A sensor system for sensing at least one compound, the sensor system comprising a sensing device comprising
- a sensor for capturing spectroscopic information regarding at least one compound in an analyte, and
- a temperature sensor for capturing temperature information regarding the analyte,
the sensor system being adapted for deriving temperature information from the spectroscopic information and for controlling and/or adapting the sensor system based on a comparison of the temperature information derived from the spectroscopic information and the temperature information captured from the temperature sensor.

2. The sensor system according to claim 1, wherein the sensor system is configured for performing, based on said comparison, an initial calibration of the sensor system after implantation or for performing a recalibration of the sensor system when in use.

3. The sensor system according to claim 2, wherein the sensor system is adapted for performing an initial calibration based on said comparison and taking into account a modelling of the sensor system behaviour after implantation.

4. The sensor system according to any of the previous claims, wherein deriving temperature information from the spectroscopic information comprises deriving temperature information from chemometrics on the spectroscopic information.

5. The sensor system according to any of the previous claims, wherein the calibration is a personalised calibration of the sensor system fit to the individual user, based on said comparison.

6. The sensor system according to any of the previous claims, wherein the sensor system is adapted for, based on said comparison, identifying a drift e.g. caused by drift of one or more components of the sensor system or drift of the sensor system caused by external causes such as encapsulation of the at least partially implantable sensing device.

7. The sensor system according to any of the previous claims, wherein the sensing device is an at least partially implantable sensing device or a fully implantable sensing device, such as for example wherein the sensing device is fully implantable subcutaneous, the sensing device being suitable for sensing at least one compound in a living creature's analyte and/or wherein the temperature sensor is integrated on a substrate or positioned on or close to a substrate in contact with the analyte.

8. The sensor system according to any of the previous claims, wherein the sensing device comprises a processor for performing said comparison.

9. The sensor system according to any of claims 1 to 7, wherein the sensor system comprises an external control device for controlling the at least partially implantable sensing device, the external control device comprising a processor for performing said comparison.

10. The sensor system according to claim 9, wherein the external control device is any of a dedicated external control device or a mobile apparatus, such as for example a mobile phone, comprising software for controlling the at least partially implantable sensing device and for performing said comparison.

11. The sensor system according to any of claims 1 to 7, wherein the sensor system is configured to communicate with a cloud-based processor for performing said comparison.

12. The sensor system according to any of the previous claims, wherein the temperature sensor is a bandgap temperature sensor, e.g. a silicon bandgap temperature sensor.

13. The sensor system according to any of the previous claims, wherein the sensor for capturing spectroscopic information is integrated in a silicon photonics integrated circuit and configured for performing spectral absorption, reflection or transmission measurements for detecting spectral information, e.g. absorption bands, of the at least one compound.

14. A method for using a sensor system for sensing at least one compound in a living creature's analyte, the method comprising
- capturing spectroscopic information using a sensor of a sensing device and deriving temperature information from said spectroscopic information,
- capturing temperature information regarding the analyte using a temperature sensor, and
controlling and/or adapting the sensor system based on a comparison of the temperature information derived from the spectroscopic information and the temperature information captured from the temperature sensor.

15. A method according to claim 14, wherein the method comprises comparing the temperature information derived from the spectroscopic information and the temperature information from the temperature sensor and deriving based thereon calibration or recalibration information fro the sensor system.
